(19) Europäisches Patentamt
European Patent Office
Office européen des brevets

(11) Publication number: **0 091 995**
**B1**

(12) **EUROPEAN PATENT SPECIFICATION**

(45) Date of publication of patent specification: **31.07.85**

(51) Int. Cl.$^4$: **C 07 C 69/612,** C 07 C 67/14, A 61 K 31/215

(21) Application number: **82112024.3**

(22) Date of filing: **27.12.82**

(54) **New compound having antiinflammatory, analgesic and mucoregulating activity, process for its production and relative pharmaceutical compositions.**

(30) Priority: **21.01.82 IT 1921682**

(43) Date of publication of application: **26.10.83 Bulletin 83/43**

(45) Publication of the grant of the patent: **31.07.85 Bulletin 85/31**

(84) Designated Contracting States: **AT BE CH DE FR GB IT LI LU NL SE**

(56) References cited: **None**

(73) Proprietor: **AUSONIA FARMACEUTICI S.r.l.**
**Via Laurentina Km. 24730**
**I-00040 Pomezia (Rome) (IT)**

(72) Inventor: **de Vincentiis, Leonardo**
**Via Isonzo 42**
**I-00198 Roma (IT)**

(74) Representative: **Bianchetti, Giuseppe**
**Studio Consulenza Brevettuale Via Senato 24**
**I-20121 Milan (IT)**

**EP 0 091 995 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to 2-methyl-5-(1-hydroxy-1-methylethyl)-cyclohex-2-enyl 2-(4-isobutylphenyl)propionate of 2-methyl-5-(1-hydroxy-1-methyl-ethyl)-cyclohexen-2-yl having the formula I

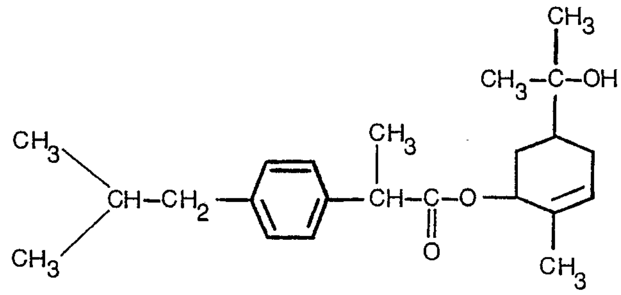

(I)

The therapeutic properties of 2-methyl-5-(1-hydroxy-1-methyl-ethyl)-2-cyclohexen-1-ol, or sobrerol (II)

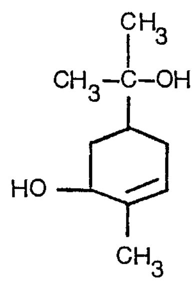

(II)

are known since many years. It has been used for the treatment of respiratory diseases (V. Dalla Valle — Boll. Chim. Farm., 109 (1970)), and more exactly as fluidifying agent with bronchosecretagogue and mucosecretolytic activity in cases of acute and chronic bronchitis with hypersecretive and obstructive component of the respiratory routes (R. Scuri et al., Il Farmaco ed. Pr. *36*, 1, (1980)).

On the other side, the antiinflammatory properties of 2-(4-isobutylphenyl)-propionic acid (Ibuprofen, see e.g. Adams et al., Pharmacodyn. Ther. *178*, 115 (1969)) are also known.

It has now unexpectedly found that the ester of the acid 2-(4-isobutylphenyl)propionic with sobrerol, that is compound I, exhibits a bronchosecretogogue activity markedly higher than that of sobrerol, and it is besides characterized by antiinflammatory and analgesic properties, comparable and partially higher than that of Ibuprofen, showing contemporaneously a gastrolesivity clearly lower than that of the latter drug.

Finally, compound (I) is also endowed with an appreciable anti-cough effect.

Thus, another object of the invention is provided by pharmaceutical compositions endowed with antiinflammatory, analgesic and mucoregolating and anticough activities, containing as active principle compound (I).

Finally, the invention relates to a process for the preparation of 2-methyl-5-(1-hydroxy-1-methylethyl)-cyclohex-2-enyl 2-(4-isobutylphenyl)propionate, characterized by reacting the chloride of 2-(4-isobutylphenyl)-propionic acid (III) with sobrerol, according to the following scheme:

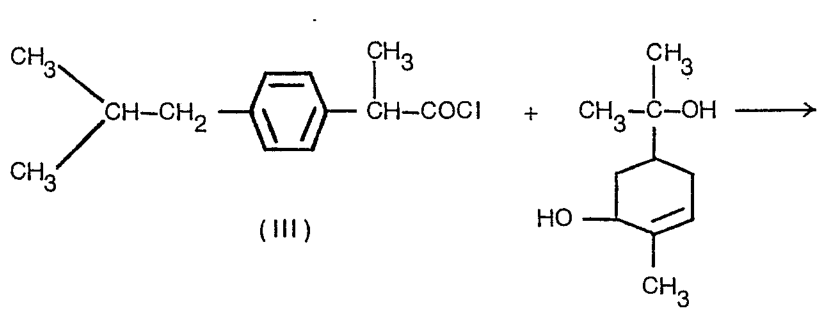

(III) + (II) → (I)

The reaction is suitably carried out in aprotic solvents, for example in ethers such as diethylether, tetrahydrofuran, dioxane, and in presence of acidity acceptors. As such tertiary bases are preferably used, such as pyridine or triethylamine. One can operate at temperatures ranging from 0 and 80°C, preferably from about 25 and 50°C.

The following example further illustrates, without limiting it, the process according to the invention.

## Example

To 55 g of chloride of 2-(4-isobutylphenyl)propionic acid and 20 ml of anhydrous pyridine 41.6 g of sobrerol (dl-transform) dissolved in 400 ml of anhydrous tetrahydrofuran are added. Immediately, precipitation of pyridine hydrochloride occurs. The reaction mixture is heated to 45°C and then, always under magentic stirring, is left at room temperature for 45 minutes. At the end the solvent is evaporated under reduced pressure, the crude residue is washed with 1 litre of water and extracted three times with diethyl ether. After having dried the organic phase on anhydrous sodium sulphate and evaporated the solvent by means of the rotary evaporator, 70 g of crude product are obtained, which is purified by Florisil chromatography (100—200 Mesh) eluting with petroleum ether, then with petroleum ether-diethyl ether 10:1.

49 Grams of a dense, yellowish oil are obtained, perfectly pure in thin layer chromatography, substantially insoluble in water, easily soluble in the common organic solvents. Its structure is confirmed by analytical and spectroscopical data.

Elemental analysis:
for $C_{23}H_{34}O_3$(M.W. = 358.49)
calcd. % C = 77.05; H = 9.56
found % C = 76.88; H = 9.60.

I.R. Spectrum
(liquid film; absorption band values are expressed in $cm^{-1}$):
stretch O—H 3400—3250
stretch C=O 1735
stretch C—O 1205

$H^1$ NMR Spectrum
(recorded in $CDCl_3$, internal reference TMS; values of the chemical shifts are expressed in δ):
0.65—1.2(m, 12H, —$CH(\underline{CH_3})_2$, $C(CH_3)_2$);
1.3—2 (m, 12H, $\underline{CH_2}$—$\underline{CH}$—$\underline{CH_2}$, $\underline{CH_2}$—$\underline{CH}$, $\underline{CH_3}$—CH, OH mobile);
2.3 (d, 3H, =C—$\underline{CH_3}$);
3.5 (q, 1H, $CH_3$—$\underline{CH}$—CO);
4.7—5 (m, 1H, —$\underline{CH}$—O—CO);
5.3—5.6 (m, 1H, =$\underline{CH}$—$CH_2$);
6.6—7 (m, 4H, aromatics).

The bio-pharmaceutical characteristics of compound (I) which will be hereinafter defined, for the sake of brevity, with the abbreviation AFP 695, have been determined as follows.

### Toxicity

The acute toxicity has been determined in Swiss mouse and in Wistar rat for different administration routes, according to the method of Litchfield J.T. and Wilcoxon [J. Pharm. Exp. Therap. *96*, 99—113, (1949)].

The results obtained are reported in the following Table I; from the same it can be pointed out how AFP is endowed with a very low acute toxicity.

TABLE I

Acute toxicity of AFP 695

| Species | Administration route | $LD_{50}$ — (mg/kg) |
|---|---|---|
| mouse | os | 1800 |
| rat | os | 2200 |

### Bronchosecretogogue activity

The bronchosecretogogue activity has been determined in male Wistar rat by per os administration according to the method of Mawatari [Mawatari H., Kagoshima Daigaku Igaku-Zasshi, *27*, 561 (1976)], comparing AFP-695 with carboxymethylcysteine and sobrerol at the doses hereinafter stated. The results are reported in the enclosed Table 2.

TABLE 2

Bronchosecretogogue activity
(Mawatari technique)

| Compound | Administered Dose mg/kg/os | No. of animals | % Increase of FlNa(*) in comparison with controls |
|---|---|---|---|
| AFP–695 | 500 | 10 | 51.6 |
| Carboxymethyl-cysteine | 500 | 10 | 39.7 |
| Sobrerol | 500 | 10 | 33.4 |

(*) FlNa = Sodium fluoresceine.

From the resulting values a more marked activity of AFP-695 in the test under exam clearly turns out in comparison with reference drugs used at equiponderant doses.

Mucosecretolytic activity

The mucosecretolytic activity has been studied in the male rabbit according to the technique of Giraldez, Grass and Bruseghini(Abstr. Comp. Int. Pharm. N. 1086-Paris 1978), by administration by the oral route, comparing AFP-695 with carboxymethylcysteine and sobrerol at the dose hereinafter stated. The results are reported in the Table 3.

TABLE 3

Mucoscretolytic activity
(technique of Giraldez et al)

| Compound | Dose mg/kg/os | No. animals | % Increase of secretion in comparison with controls |
|---|---|---|---|
| AFP–695 | 500 | 10 | 35.7 |
| Carboxymethyl-cysteine | 500 | 10 | 40.2 |
| Sobrerol | 500 | 10 | 36.8 |

From the comparison of the results obtained by the test under exam the mucosecretolytic activity of AFP-695 results comparable to that of carboxymethylcysteine and sobrerol used at equiponderant doses.

Antiflammatory activity

a) *Carrageenin edema*

Tests have been performed on the rat, employing the test of the subplantar edema induced by carrageenin. AFP-695 and, for comparison, ibuprofen, have been administered by oral route 30 minutes before carrageenin, at the doses hereinafter stated. After edema induction, the animals of each group have been controlled, every 60 minutes for 5 consecutive hours, in order to point out the evolution of the inflammatory process.

The results obtained, expressed as volume of edematous limbs, are reported in the Table 4. From the comparison between the values of control rats and those of AFP-695 treated rats, it turns out the very good inflammatory activity of the latter, comparable to that of ibuprofen, in the used experimental conditions and at the used doses.

TABLE 4

Antiinflammatory activity – subplantar edema by carrageenin in the rat

| Treatment | Dose mg/kg os | MEAN LIMB VOLUME AT DIFFERENT HOURS AFTER TREATMENT | | | | | | AREA | |
|---|---|---|---|---|---|---|---|---|---|
| | | 0 | 1 | 2 | 3 | 4 | 5 | Absolute value | % Inhibition in comparison with controls |
| Controls | – | 23.8 | 30.7 | 35.1 | 40.2 | 43.7 | 44.8 | 273.1 | – |
| Ibuprofen | 100 | 22.5 | 26.7 | 28.9 | 29.8 | 32.4 | 35.5 | 152.4 | 44.2 |
| AFP–695 | 150 | 22.8 | 27.2 | 29.6 | 31.4 | 32.6 | 34.9 | 156.2 | 42.8 |

b) *Pleuritis by carrageenin*

It has been used the method described by Vinegar and Co-workers (Vinegar R., Irvaux J.F., Selph J.I. proc. Soc. Exp. Biol. Med. *143*, 3 (1973). Sprague-Dawley rats of both sexes, weighing 150—200 g, have been used. The animals have been divided in groups "at random" and left fasting since the preceding day, with water "ad libitum." The compounds under exam have been administered by the oral route, 30 minutes before of the intrapleural injection, at the doses hereinafter stated. The results are described in enclosed Table 5.

TABLE 5

Pleuritis by carrageenin

| Treatment | Doses mg/kg os | No. animals | Hours | Volume | % Inhibition in comparison with controls | % Leucocytes × 1000 | % Inhibition in comparison with controls |
|---|---|---|---|---|---|---|---|
| Controls | | 10 | 5 | 0.98 | = | 17.120 | = |
| | | | 24 | 0.90 | = | 18.460 | = |
| AFP–695 | 100 | 10 | 5 | 0.57 | 41.84 | 4.970 | 70.91 |
| | | | 24 | 0.48 | 51.02 | 7.220 | 60.89 |
| Ibuprofen | 100 | 10 | 5 | 0.54 | 44.90 | 4.420 | 74.18 |
| | | | 24 | 0.79 | 19.39 | 17.430 | 5.58 |

From the comparison of the results it turns out that AFP-695 causes a 41.84% decrease of the exudate (provoked by pleuritis by carrageenin) at the fifth hour after animals' treatment, and a 70.91% decrease of the number of leucocytes present at the same hour (decreases of the same order of magnitude than those provoked by the reference drug, used at equiponderant doses).

At the $24^{th}$ hours AFP-695 exerts a protective action by far higher than that of ibuprofen: in fact AFP-695 yet exerts a 51.02% decrease of the exudate volume, in comparison with control, and a 60.89% decrease of leucocytes number, whilst the reference drug decreases the exudate volume only of 19.39% and leucocytes number of 5.58%.

Such an experiment, considering that the two drugs have been used at equiponderant doses and not equimolar, and taking into account, besides to the volume of pleuric exudate, also the count of leucocytes present, shows an antiinflammatory activity higher in absolute way and of longer duration of AFP-695 in comparison with the reference drug. Such an higher pharmacological activity is probably due to AFP-695 pulmonaty tropism shown by the pharmacokinetics study (see following pages).

Gastrolesive action

Rats fasting since 18 hours have been treated by oral route with AFP-695 and, for comparison, with Ibuprofen, at the doses hereinafter stated. 5 Hours and 30 minutes after treatments, the animals have been sacrificed and the stomach has been withdrawn for gastric mucosa examination.

As it is possible to evidence from the results obtained, reported in the Table 6, the gastrolesivity of AFP-695, in the used experimental conditions, is by far lower than that of the reference drugs.

TABLE 6

Gastrolesive action

| Treatment | Dose mg/kg/os | Mean ulcers size (mm) |
|---|---|---|
| Controls | – | 0 |
| Ibuprofen | 100 | 9.6 ± 1.8 |
| AFP–695 | 150 | 0.9 ± 0.3 |

Analgesic activity

The test of writhings by phenylquinone in the mouse has been used. AFP-695 has been administered by oral route at the dose of 100 mg/kg in comparison with ibuprofen at the same doses.

The two drugs have been administered per os 30 minutes after intraperitoneal injection of phenylquinone.

The very good analgesic activity of AFP-695, slightly lower than that of ibuprofen administered at equiponderant doses, is shown from the results obtained and reported in the Table 7.

TABLE 7

Analgesic activity — Writhing by phenyl quinone in the mouse

| Treatment | Dose mg/kg os | Mean No. of writhings | % Inhibition in comparison with controls | No. animals with writhings |
|---|---|---|---|---|
| Controls | – | 12.8 ± 2.6 | — | 10/10 |
| Ibuprofen | 100 | 1.3 ± 0.5 | 89.84 | 1/10 |
| AFP–695 | 100 | 3.7 ± 1.2 | 71.09 | 3/10 |

Anti-cough activity

The anti-cough activity of AFP-695 has been assessed after administration by endoperitoneal route (30% in tween) on rabbits of both sexes, weighing 200—350 g, according to the method of the cough

induced by citric acid aerosol administration, and evaluating the mean number of cough strokes in comparison with the same basal values (Boura and coll. — Prog. Brit. Pharmac. Soc. Apr. 1970). As reference standard a group of codeine treated animals at the dose of 12.5 mg/kg has been used.

TABLE 8

Anti-cough activity

| Treatment | Dose mg/kg | No. animals | % Inhibition in comparison with controls | Statistical significance (basal minus final values) |
|---|---|---|---|---|
| Controls | – | 12 | 26.7 | – |
| Codeine | 12.5 | 12 | 76.7 | $p < 0.05$ |
| AFP–695 | 150 | 12 | 78.1 | $p < 0.05$ |

From the above stated results it becomes evident the very good anti-cough activity of AFP-695 in comparison with traditional standard of proved activity, in the used experimental conditions.

Pharmacokinetics

Two groups of rats have been treated per os. by gastic tube, with AFP-695 at the dose of 100 mg/kg; as a reference standard ibuprofen at the dose of 58 mg/kg has been used. At the moment of treatment rats were fasting since 18 hours. Rats have been then sacrificed in groups of 4 at different times: 30 min., 1 h, 2 h, 4 h, 6 h.

The concentration both of unchanged products and of their metabolites have been measured by gas chromatography in plasma and in the lung. At 30 minutes, and in some instance also at 1 hour, unchanged AFP-695 has been found in plasma showing that this product is at least partially absorbed as such.

The plasma levels of Ibuprofen proved to be higher after administration of AFP-695 in comparison with the administration of Ibuprofen at equimolar doses. The difference (about 15%) was more marked in the first two hours and it is relative to a better bioavailability for Ibuprofen. Also pulmonary levels of Ibuprofen turned out higher after administration of AFP-695. The concentrations rations between lung and plasma in case of Ibuprofen were 0.6 for Ibuprofen administration and 0.8 for AFP-695 administration.

Compound (I) is therefore fit as an elective drug with antiinflammatory, analgesic, mucoregulating and anti-cough activities, in the therapy of acute and chronic tracheo-broncho-pulmonary diseases on inflammatory basis associated with catarrh, cough and algic synthomathology.

The present invention refers also to all the industrially applicable aspects connected with the use of AFP-695 as antiinflammatory, analgesic, mucoregulating and anti-cough agent. An essential aspect of the invention is therefore provided by pharmaceutical compositions containing predetermined amounts of AFP-695. Such a compound can be administered, particularly, by the oral route, as capsules, extemporary suspension, monodose sachets (in a granular state); or by aerosol, or as suppositories; or, finally, by parenteral route (vials for injection).

Of course, the single pharmaceutical forms will contain the active principle together with veichles, excipients, flavouring agents etc. of conventional use in pharmaceutical technique.

**Claims for the Contracting States: BE CH DE FR GB IT LI LU NL SE**

1. A compound, 2-methyl-5-(1-hydroxy-1-methylethyl)-cyclohex-2-enyl 2-(4-isobutylphenyl)propionate having the formula I

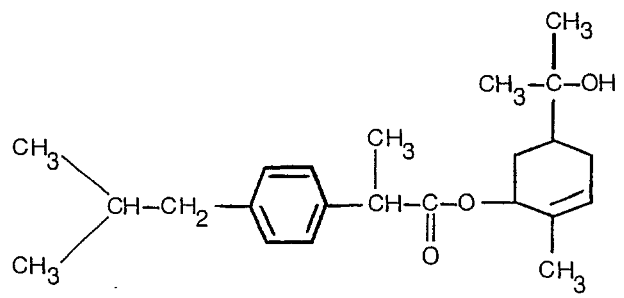

(I)

2. Pharmaceutical compositions endowed with antiinflammatory, bronchosecretogogue, analgesic, mucoregulating and anti-cough activity, characterized by containing as active principle the compound of claim 1.

3. Process for the preparation of 2-methyl-5-(1-hydroxy-1-methylethyl)-cyclohex-2-enyl 2-(4-isobutylphenyl)propionate, having formula I, characterized by reacting the chloride of 2-(4-isobutylphenyl)propionic acid (III) with sobrerol (II), according to the following scheme:

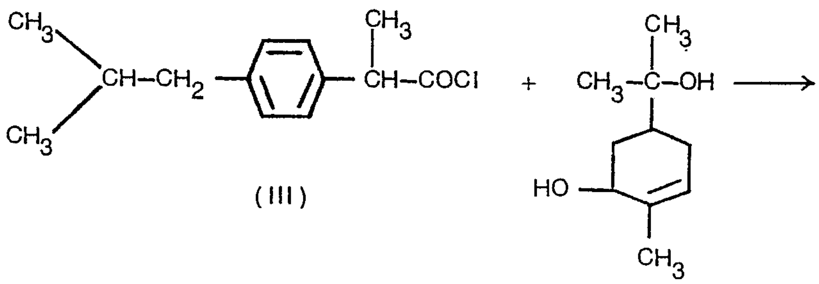

(III) (II) (I)

4. Process according to claim 3, characterized by operating in inert solvents, such as diethylether, tetrahydrofuran or dioxan.

5. Process according to claims 3—4, characterized by operating in the presence of acidity acceptors, such as triethylamine or pyridine.

6. Process according to claims 3—5, characterized by operating at temperatures ranging from about 0 to 80°C, preferably from 25 to 50°C.

**Claims for the Contracting State: AT**

1. Process for the preparation of 2-methyl-5-(1-hydroxy-1-methylethyl)-cyclohex-2-enyl 2-(4-isobutylphenyl)propionate having the formula I

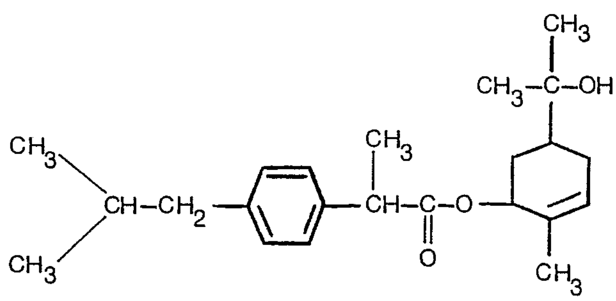

(I)

characterized by reacting the chloride of 2-(4-isobutylphenyl)propionic acid (III) with sobrerol (II), according to the following scheme:

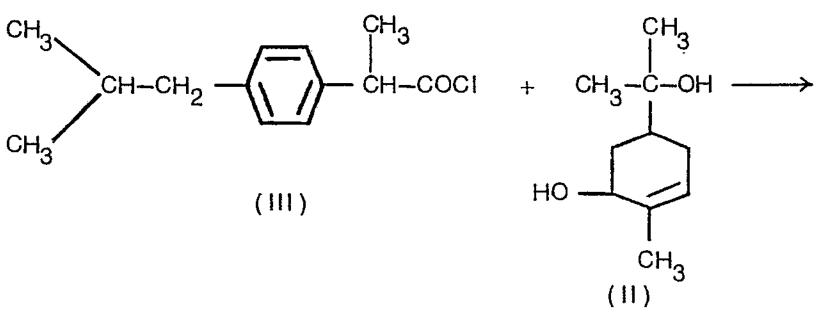

(III) (II) (I)

2. Process according to claim 1, characterized by operating in inert solvents, such as diethylether, tetrahydrofuran or dioxan.

3. Process according to claims 1—2, characterized by operating in the presence of acidity acceptors, such as triethylamine or pyridine.

4. Process according to claims 1—3, characterized by operating at temperatures ranging from about 0 to 80°C, preferably from 25 to 50°C.

**Patentansprüche für die Vertragsstaaten: BE CH DE FR GB IT LI LU NL SE**

1. Eine Verbindung 2-Methyl-5-(1-hydroxy-1-methylethyl-cyclohex-2-enyl 2-(4-isobutylphenyl)-propionat mit der Formel I

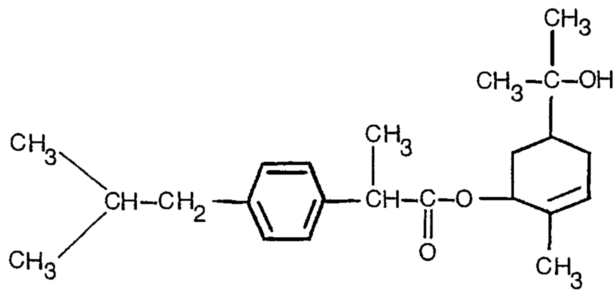

(I)

2. Pharmazeutische Zusammensetzungen ausgestattet mit entzündungshemmender, bronchialschleimbefördernder, schmerzstillender, schleimregulierender und hustenhemmender Wirkung dadurch gekennzeichnet, daß sie als aktiven Bestandteil die Verbindung aus Anspruch 1 enthalten.

3. Verfahren zur Herstellung von 2-Methyl-5-(1-hydroxy-1-methylethyl)-cyclohex-2-enyl 2-(4-isobutylphenyl)-propionat, mit der Formel I, dadurch gekennzeichnet, daß 2-(4-isobutylphenyl) Propionylchlorid (III) mit Sobrerol (II) zur Reaktion gebracht wird entsprechend dem folgenden Schema.

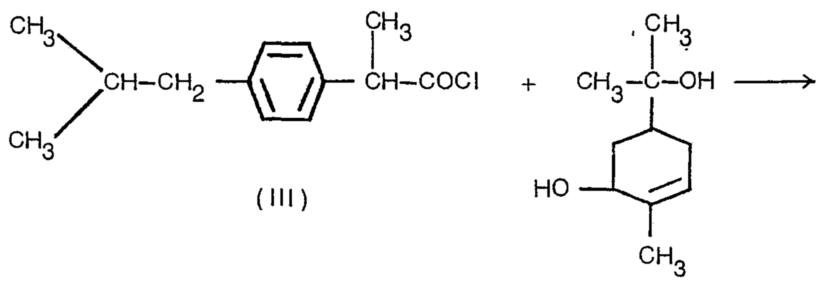

(III) + (II) → (I)

4. Verfahren gemäß Anspruch 3 dadurch gekennzeichnet, daß in inerten Lösungsmitteln gearbeitet wird, solche wie Diethyläther, Tetrahydrofuran oder Dioxan.

5. Verfahren gemäß den Ansprüchen 3—4 dadurch gekennzeichnet, daß in Gegenwart von Säurebindern gearbeitet wird, solche wie Triethylamin oder Pyridin.

6. Verfahren gemäß den Ansprüchen 3—5 dadurch gekennzeichnet, daß bei Temperaturen im Bereich zwischen 0—80 Grad C, vorzugsweise zwischen 25 und 50 Grad C gearbeitet wird.

**Patentansprüche für den Vertragsstaat: AT**

1. Verfahren zur Herstellung von 2-Methyl-5-(1-hydroxy-1-methylethyl-cyclohex-2-enyl 2-(4-isobutylphenyl)propionat mit der Formel I

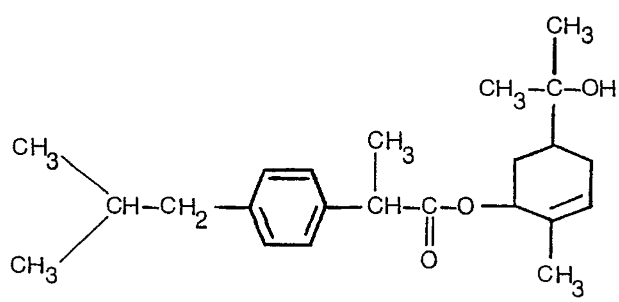

(I)

dadurch gekennzeichnet, daß 2-(4-isobutylphenyl)Propionylchlorid (III) mit Sobrerol (II) zur Reaktion gebracht wird, entsprechend dem folgenden Schema:

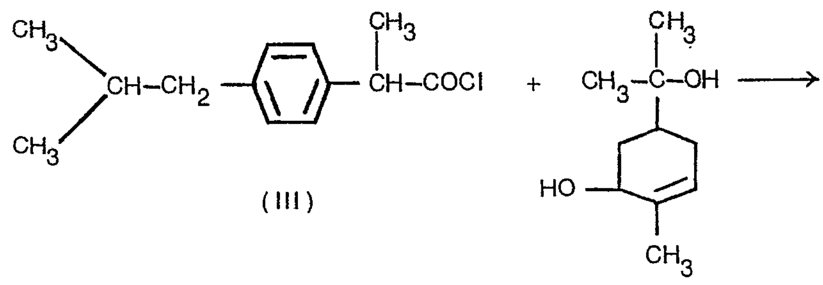

(III) (II) (I)

2. Verfahren gemäß Anspruch 1 dadurch gekennzeichnet, daß in inerten Lösungsmitteln gearbeitet wird, solche wie Diethyläther, Tetrahydrofuran oder Dioxan.

3. Verfahren gemäß den Ansprüchen 1—2 dadurch gekennzeichnet, daß in Gegenwart von Säurebindern gearbeitet wird, solche wie Triethylamin oder Pyridin.

4. Verfahren gemäß den Ansprüchen 1—3 dadurch gekennzeichnet, daß bei Temperaturen im Bereich von 0—80 Grad C vorzugsweise zwischen 25 und 50 Grad C gearbeitet wird.

**Revendications pour les Etats contractants: BE CH DE FR GB IT LI LU NL SE**

1. Un composé, le 2-(4-isobutylphényl)-propionate de 2-méthyl-5-(1-hydroxy-1-méthyléthyl)-cyclohex-2-ényle, ayant la formule I:

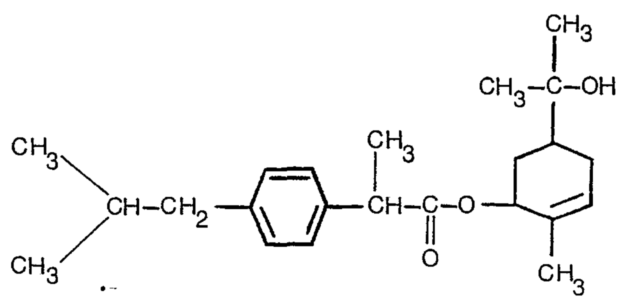

(I)

2. Compositions pharmaceutiques ayant une activité anti-inflammatoire, bronchosécrétogogue, analgésique, mucorégulatrice et anti-tussive, caractérisées en ce qu'elles contiennent, comme principe actif, le composé de la revendication 1.

3. Procédé de préparation de 2-(4-isobutylphényl)-propionate de 2-méthyl-5-(1-hydroxy-1-méthyléthyl)-cyclohex-2-ényle ayant la formule I, caractérisé en ce qu'on fait réagir le chlorure de l'acide 2-(4-isobutylphényl)propionique (III) avec du sobrérol (II), selon la schéma suivant:

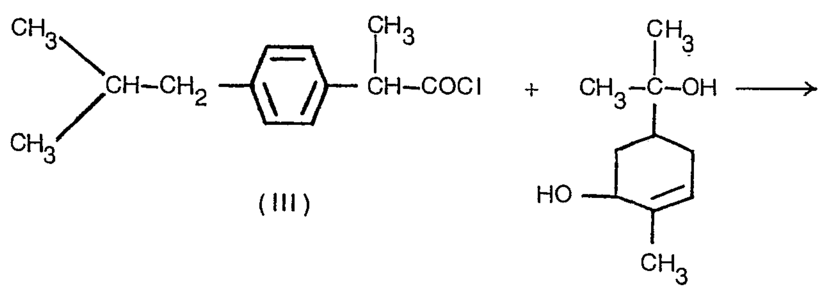

(III) (II) (I)

4. Procédé selon la revendication 3, caractérisé en ce qu'on opère dans des solvants inertes, tels que l'éther diéthylique, le tétrahydrofuranne ou le dioxanne.

5. Procédé selon les revendications 3—4, caractérisé en ce qu'on opère en présence d'accepteurs d'acide, tels que la triéthylamine ou la pyridine.

6. Procédé selon les revendications 3—5, caractérisé en ce qu'on opère à des températures allant d'environ 0 à 80°C, de préférence de 25 à 50°C.

**Revendications pour l'Etat contractant: AT**

1. Procédé de préparation de 2-(4-isobutylphényl)-propionate de 2-méthyl-5-(1-hydroxy-1-méthyléthyl)-cyclohex-2-ényle ayant le formule I

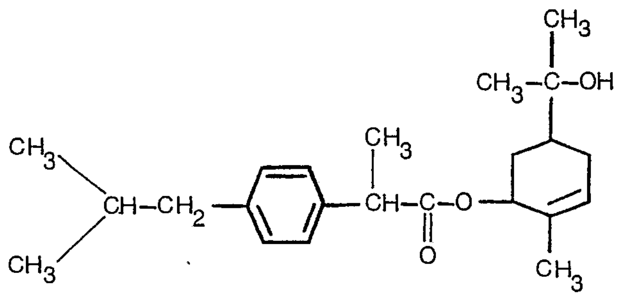

(I)

caractérisé en ce qu'on fait réagir le chlorure de l'acide 2-(4-isobutylphényl)propionique (III) avec du sobrérol (II) selon le schéma suivant:

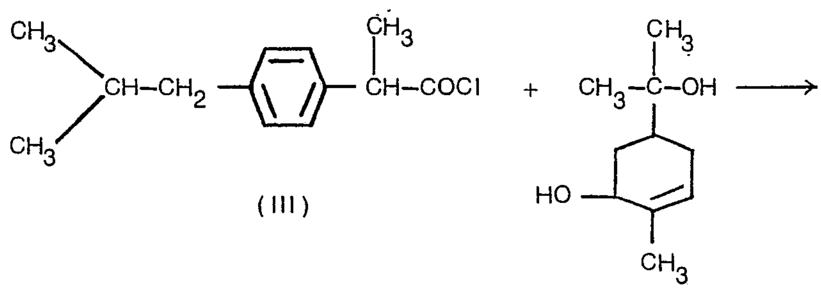

(III)

(II)

(I)

2. Procédé selon la revendication 1, caractérisé en ce qu'on opère dans des solvants inertes, tels que l'éther diéthylique, le tétrahydrofuranne ou le dioxanne.

3. Procédé selon les revendications 1—2, caractérisé en ce qu'on opère en présence d'accepteurs d'acide, tels que la triéthylamine ou la pyridine.

4. Procédé selon les revendications 1—3, caractérisé en ce qu'on opère à des températures allant d'environ 0 à 80°C, de préférence de 25 à 50°C.